(19) Europäisches Patentamt
European Patent Office
Office européen des brevets

(11) **EP 3 276 007 A1**

(12) **EUROPEAN PATENT APPLICATION**
published in accordance with Art. 153(4) EPC

(43) Date of publication:
**31.01.2018 Bulletin 2018/05**

(51) Int Cl.:
*C12P 7/64* [(2006.01)]   *A23L 33/12* [(2016.01)]
*A61K 9/48* [(2006.01)]   *A61K 31/232* [(2006.01)]
*A61K 47/14* [(2017.01)]   *A61K 47/44* [(2017.01)]
*A61P 3/02* [(2006.01)]   *A61P 3/06* [(2006.01)]
*A61P 7/02* [(2006.01)]   *A61P 25/28* [(2006.01)]

(21) Application number: **16768968.6**

(22) Date of filing: **25.03.2016**

(86) International application number:
**PCT/JP2016/059770**

(87) International publication number:
**WO 2016/153065 (29.09.2016 Gazette 2016/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.03.2015  JP 2015063221**

(71) Applicant: **Kewpie Corporation**
**Tokyo 150-0002 (JP)**

(72) Inventors:
• **KOBAYASHI, Hideaki**
  **Tokyo 182-0002 (JP)**
• **HOSHINA, Ryosuke**
  **Tokyo 182-0002 (JP)**
• **KATAGIRI, Kazumi**
  **Tokyo 182-0002 (JP)**

(74) Representative: **J A Kemp**
**14 South Square**
**Gray's Inn**
**London WC1R 5JJ (GB)**

(54) **METHOD FOR PRODUCING DHA-CONTAINING GLYCERIDE-CONTAINING COMPOSITION**

(57)    A method for producing a composition containing a DHA-containing glyceride, including a step of obtaining a composition containing a DHA-containing glyceride, in which an esterification reaction is conducted by reacting a lipase with a raw material oil and fat containing highly unsaturated fatty acid as a constituent fatty acid in the presence of water and a lower alcohol to give a composition containing a DHA-containing glyceride, wherein a reaction liquid in the reaction has a water content of 0.4% by mass or more, and wherein the composition containing a DHA-containing glyceride has an acid value of 12 or less.

## Description

### Technical Field

The present invention relates to a method for producing a composition containing a DHA-containing glyceride having high purity and fine quality, and to a composition containing a DHA-containing glyceride.

### Background Art

Highly unsaturated fatty acids and derivatives thereof have many biological activities such as decreasing of fat in blood, and have been used as raw materials for pharmaceuticals, cosmetic preparations and foods since before. It is known that, among these, specifically docosahexaenoic acid (DHA, C22 : 6) has various biological activities such as an action to suppress flocculation of platelets, an action to lower blood neutral fat, an action to lower blood cholesterol and an effect to improve cerebral functions, and is very high in demand in Japan and foreign countries as pharmaceuticals, specified health foods, foods with function claims, supplements and the like.

As DHA ethyl esters, those having high purity are commercially available, and it is known that a glyceride form has more excellent absorption in the body than that of an ethyl ester form. Therefore, a method for producing a composition containing a DHA-containing glyceride having high purity and fine quality is required.

For example, Patent Literature 1 (Japanese Examined Patent Publication No. H7-87793) describes a method for obtaining a monoglyceride containing a highly unsaturated fatty acid and a free fatty acid by hydrolyzing a raw material oil and fat with a lipase, and further fractionating the product by chromatography.

However, the monoglyceride obtained by the above-mentioned method also contains other unsaturated fatty acids such as EPA besides DHA, and thus selective concentration of DHA has not been attained. Furthermore, since free fatty acids are generated in large amounts, the acid value increases, and thus a DHA-containing monoglyceride having high purity and fine quality cannot be obtained.

Furthermore, Patent Literature 2 (Japanese Patent No. 453031) describes a method including: Step a, in which a raw material oil and fat is reacted with a lipase, and subjected to a distillation treatment to separate a glyceride fraction comprising DHA and EPA; and subsequent Step b, in which the glyceride fraction is reacted with a lipase to thereby give a glyceride fraction in which DHA has been concentrated.

However, in the above-mentioned method, similarly to the method described in Patent Literature 1, the obtained glyceride fraction has a high free fatty acid content, and thus a DHA-containing monoglyceride having high purity and fine quality cannot be obtained. Furthermore, it is necessary to undergo two enzyme reaction steps, and thus the loss of the intended product is large, and a sufficient amount of DHA-containing glyceride cannot be obtained.

### Citation List

### Patent Literature

    Patent Literature 1: Japanese Examined Patent Publication No. H7-87793
    Patent Literature 2: Japanese Patent No. 453031

### Summary of Invention

### Problems to be Solved by the Invention

Therefore, the object of the present invention is to provide a production method by which a composition containing a DHA-containing glyceride having high purity and fine quality can be efficiently obtained, and a composition containing a DHA-containing glyceride.

### Means for Solving the Problems

The present inventors found that, in a step of conducting an esterification reaction by reacting a lipase with a raw material oil and fat containing a highly unsaturated fatty acid, if the water content in the reaction liquid of the above-mentioned reaction is controlled so as to achieve the above-mentioned object, a composition containing a DHA-containing glyceride having a low acid value (high purity and fine quality) can be unexpectedly obtained, and consequently completed the present invention.

That is, the present invention is as follows.

(1) A method for producing a composition containing a DHA-containing glyceride, comprising:

a step of obtaining a composition containing a DHA-containing glyceride, in which an esterification reaction is conducted by reacting a lipase with a raw material oil and fat containing highly unsaturated fatty acids as constituent fatty acids in the presence of water and a lower alcohol to give a composition containing a DHA-containing glyceride,
wherein a reaction liquid in the reaction has a water content of 0.4% by mass or more, and
wherein the composition containing a DHA-containing glyceride has an acid value of 12 or less.

(2) The method for producing a composition containing a DHA-containing glyceride according to (1),
wherein the reaction liquid contains 0.1 part by mass or more and 3.0 parts by mass or less of the lower alcohol with respect to 9.5 parts by mass of the raw material oil and fat.

(3) The method for producing a composition containing a DHA-containing glyceride according to (1) or (2),
wherein the proportion of the lower alcohol fatty acid esterified product with respect to the composition containing a DHA-containing glyceride is 40% by mass or more.

(4) A composition containing a DHA-containing glyceride having an acid value of 12 or less,
having a glyceride content of 20% by mass or more and 60% by mass or less with respect to the oil and fat components in the composition containing a DHA-containing glyceride, and
containing DHA and EPA as constituent fatty acids in the glycerides at the following mass ratio:

$$0.5 \leq (\mathrm{DHA} / \mathrm{EPA\ in\ glycerides}) \leq 10.$$

(5) The composition containing a DHA-containing glyceride according to (4),
wherein the proportion of the DHA contained as the constituent fatty acid in the glycerides of the composition containing a DHA-containing glyceride is 20% by mass or more and 90% by mass or less.

(6) The composition containing a DHA-containing glyceride according to (4) or (5),
wherein the composition containing a DHA-containing glyceride contains a monoglyceride at a ratio of 1% by mass or more and 55% by mass or less and a diglyceride at a ratio of 1% by mass or more and 35% by mass or less with respect to the oil and fat components in the composition containing a DHA-containing glyceride.

(7) The method for producing a composition containing a DHA-containing glyceride according to any one of (1) to (3),
wherein the composition obtained by the esterification reaction further contains a lower alcohol fatty acid ester,
the method further comprises a step of separating a glyceride and other components by molecular distillation of the composition containing a DHA-containing glyceride, and
wherein the glyceride content of the composition containing a DHA-containing glyceride is 70% by mass or more and 100% by mass or less.

(8) A composition containing a DHA-containing glyceride having an acid value of 12 or less,
having a glyceride content of 70% by mass or more and 100% by mass or less with respect to the oil and fat components in the composition containing a DHA-containing glyceride, and
containing DHA and EPA as constituent fatty acids in the glycerides at the following mass ratio:

$$0.5 \leq (\mathrm{DHA} / \mathrm{EPA\ in\ glycerides}) \leq 10.$$

(9) The composition containing a DHA-containing glyceride according to (8),
wherein the proportion of the DHA contained as the constituent fatty acid in the glycerides of the composition containing a DHA-containing glyceride is 20% by mass or more and 90% by mass or less.

(10) The composition containing a DHA-containing glyceride according to (8) or (9), which contains a monoglyceride at a ratio of 5% by mass or more and 45% by mass or less and a diglyceride at a ratio of 30% by mass or more and 90% by mass or less with respect to the oil and fat components in the composition containing a DHA-containing glyceride.

(11) The method for producing a composition containing a DHA-containing glyceride according to (7),
wherein the method further comprises a step of conducting a hydrolysis reaction by reacting a lipase with the composition obtained by the molecular distillation treatment in the presence of water, and further separating a glyceride and other components by molecular distillation, and

wherein the proportion of the DHA contained as the constituent fatty acid in the glycerides of the composition containing a DHA-containing glyceride is 30% by mass or more and 80% by mass or less.

(12) A composition containing a DHA-containing glyceride having an acid value of 0.1 or more and 5 or less, having a glyceride content of 80% by mass or more and 100% by mass or less with respect to the oil and fat components in the composition containing a DHA-containing glyceride, and

containing DHA and EPA as constituent fatty acids in the glycerides at the following mass ratio:

$$2 \leq (\text{DHA} / \text{EPA in glycerides}) \leq 15.$$

(13) The composition containing a DHA-containing glyceride according to (12),

wherein the proportion of the monoglyceride is 0% or more and 20% by mass or less, and the proportion of the diglyceride is 5% by mass or more and 50% by mass or less with respect to the oil and fat components in the composition containing a DHA-containing glyceride.

(14) The composition containing a DHA-containing glyceride according to (12) or (13),

wherein the proportion of the triglyceride with respect to the oil and fat components in the composition containing a DHA-containing glyceride is 40% or more and 95% by mass or less.

(15) A composition containing a DHA-containing glyceride obtained by the production method according to any one of (1) to (3), (7) and (11).

(16) A method for producing a food composition, comprising a step of obtaining a food composition by using a composition containing a DHA-containing glyceride obtained by the production method according to any one of (1) to (3), (7) and (11), or the composition containing a DHA-containing glyceride according to any one of (4) to (6), (8) to (10) and (12) to (15).

(17) A method for producing a capsule formulation, comprising a step of obtaining an capsule formulation by using a composition containing a DHA-containing glyceride obtained by the production method according to any one of (1) to (3), (7) and (11), or the composition containing a DHA-containing glyceride according to any one of (4) to (6), (8) to (10) and (12) to (15).

**Effects of the Invention**

The present invention can provide a production method by which a composition containing a DHA-containing glyceride having high purity and fine quality can be efficiently obtained, and thus contributes to further expansion of foods, pharmaceuticals, forages and the like utilizing a DHA-containing glyceride.

Furthermore, since the stability of the lipase can be increased by controlling the water content in the reaction liquid for the esterification reaction, the lipase can be repetatively used, and thus the production cost can be decreased and the resource can be saved.

**Brief Description of Drawings**

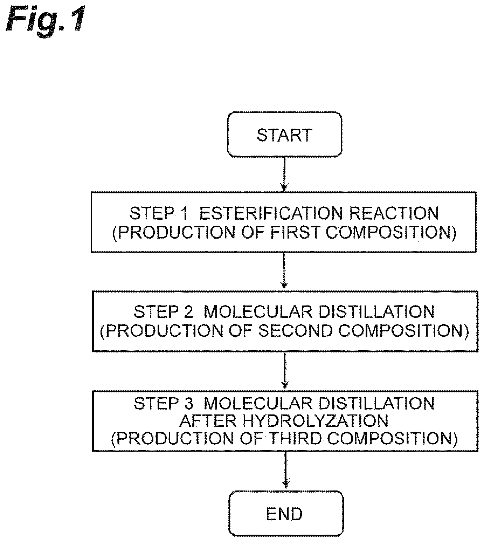

FIG. 1 shows a flow chart of the method for producing a composition containing a DHA-containing glyceride of the present invention.

**Embodiments for Carrying Out the Invention**

The present invention will be explained in detail. In the present invention, "%" means "% by mass", and "part(s)" means "part(s) by mass".

<Feature of Present Invention>

The production method of the present invention is characterized by a production method for efficiently obtaining a composition containing a DHA-containing glyceride having high purity and fine quality, including a step of obtaining a composition containing a DHA-containing glyceride by conducting an esterification reaction by reacting a lipase with a raw material oil and fat containing highly unsaturated fatty acid as a constituent fatty acid in the presence of water and a lower alcohol, wherein a reaction liquid in the reaction has a water content of 0.4% by mass or more, and the composition containing a DHA-containing glyceride has an acid value of 12 or less.

More specifically, a DHA-containing glyceride can be selectively produced by concentrating DHA in a glyceride fraction by the production method of the present invention.

<DHA-Containing Glyceride>

In the present invention, the glyceride refers to glycerin fatty acid esters including a monoglyceride, a diglyceride and a triglyceride. Furthermore, the DHA-containing glyceride refers to a compound in which a part or whole part of the fatty acid residues that constitute a glycerin fatty acid esterified product is DHA.

<Raw Material Oil and Fat>

In the production method of the present invention, an oil and fat generally means a triglyceride, but in the present invention, the oil and fat may also include other glycerides that react with a lipase such as a diglyceride and a monoglyceride. Furthermore, the raw material oil and fat used in the production method of the present invention may be any oil and fat containing a triglyceride containing a highly unsaturated fatty acid containing DHA as a constituent fatty acid. Specifically, in the esterification reaction, it is preferable to use an oil and fat containing a triglyceride at a ratio of 90% by mass or more.
In the present invention, "the oil and fat component in the composition containing a DHA-containing glyceride" refers to a concept encompassing triglycerides, diglycerides, monoglycerides, lower alcohol esterified products and free fatty acids.

<Composition of Raw Material Oil and Fat>

As the raw material oil and fat used for the production method of the present invention, it is preferably to use an oil and fat having a DHA content a fatty acid composition of 2% by mass or more and 40% by mass or less (preferably 2% by mass or more and 35% by mass or less). Furthermore, they may contain a triglyceride containing fatty acids other than DHA such as EPA (eicosapentaenoic acid, C20 : 5) as constituent fatty acids may also be contained.
In the case when the raw material oil and fat contains EPA as a constituent fatty acid, the content of the EPA in the fatty acid composition can be 10% by mass or more and 30% by mass or less (preferably 10% by mass or more and 20% by mass or less). In the case when the raw material oil and fat contains both EPA and DHA, since the DHA content in the glyceride in the obtained composition containing a DHA-containing glyceride can further be increased, and thus the mass ratio of the EPA and DHA is preferably EPA/DHA = 0.5 or more and 6 or less, further preferably 0.5 or more and 3 or less.
Furthermore, since the acid value of the obtained composition containing a DHA-containing glyceride can be decreased easily, the acid value of the raw material oil and fat is preferably 0 or more and 2.5 or less, and is further preferably 0 or more and 2 or less.

<Kind of Raw Material Oil and Fat>

Examples of the raw material oil and fat include fish oils, animal oils other than fish oils, vegetable oils, oils produced by algae or microorganisms, or mixed oil and fat thereof, and the like.
Examples of the fish oils include sardine oil, tuna oil, bonito oil, codfish liver oil, salmon oil, squid oil, or menhaden oil, and the like.
Examples of the oils derived from algae or microorganisms include, for example, arachidonic acid-containing oils derived from Mortierella alpine, Euglena gracilis and the like, EPA-containing oils derived from Ecklonia kurome, Eisenia bicyclis, brown seaweed, Hizikia fusiforme, Petalonia binghamiae, a certain kind of F. evanescens, DHA-containing oils by Crypthecodinium cohnii, Vibrio marinus, Thraustochytrium aureum, Shewanella bacteria and the like.

<Lipase>

The lipase used in the production method of the present invention (an esterification reaction) may be either 1,3-positions specific or non-specific. Examples of the 1,3-positions specific lipase is an enzyme that specifically acts on only the 1,3-positions of a triglyceride, or an enzyme that preferentially acts on 1,3-positions rather than on the 2-position. Since the proportion of the composition containing a DHA-containing glyceride can further be increased, the 1,3-positions specific lipase is preferably used.
The characteristic of the lipase may be either roughly-purified, partially purified or purified. Furthermore, the lipase may be either a free form or an immobilized enzyme, and it is preferable to use an immobilized enzyme in that it can be recycled, and the treatment after the esterification reaction is easy.

<Support>

The immobilized enzyme may be one obtained by immobilizing an enzyme on a support, a film or a membrane. Examples of the support include organic supports, inorganic supports and organic-inorganic composite supports such as ion exchange resins, porous resins, ceramics, calcium carbonate, celite, glass beads and active carbon. It is preferable to use an ion exchange resin, a porous resin or a ceramic as the support since they are excellent in durability and affinity to the lipase.

Examples of the method for the immobilization include an inclusion process, a crosslinking process, a physical adsorption process, an ion adsorption process, a covalent bonding process, a hydrophobic bonding process and the like.

<Particle Diameter>

In the case when the immobilized enzyme has a particulate form, an enzyme can be immobilized on the particulate support. In this case, the particle diameter of the support may be 0.01 mm or more and 3 mm or less, and may further be 0.05 mm or more and 1.5 mm or less.

<Specific Examples of Lipase>

In the esterification reaction of the present invention, examples of the lipase include filamentous fungi belonging to Rhizomucor (Rhizomucor miehei), Mucor (Mucor miehei, Mucor javanicus), Aspergillus (Aspergillus oryzae, Aspergillus niger), Rhizopus (Rhizopus sp.), Penicillium (Penicillium roqueforti, Penicillium camemberti), Thermomyces (Thermomyces lanuginosus) and the like; yeasts belonging to Candida (Candida antarctica, Candida rugosa, Candida cylindracea), Pichia (Pichia) and the like; bacteria belongs to Pseudomonas (Pseudomonas sp.), Achromobacter (Achromobacter sp.), Burkholderia (Burkholderia sp.), Alcaligenes (Alcaligenes sp.), Pseudozyma (Pseudozyma sp.) and the like; and lipases derived from animals such as swine liver. For example, lipases derived from Rhizopus oryzae (lipase DF: manufactured by Amano Enzyme, Inc.) and Candida rugosa (lipase OF: manufactured by Meito Sangyo Co., Ltd.) and lipases from Pseudomanas (lipase PS, lipase AK: manufactured by Amano Pharmaceutical Co., Ltd.) are exemplified, and examples of the immobilized enzymes include a lipase from Rhizomucor miehei (LypoZyme IM60: manufactured by Novozymes, LypoZyme RMIM: manufactured by Novozymes) and a lipase from Pseudozyma antarctica (Novozyme 435: manufactured by Novozymes).

<Use Amount of Lipase>

The use amount of the lipase is not specifically limited as long as the esterification reaction progresses. For example, in the case when an enzyme having a free form is used, the lipase generally can be added in 1 unit (U) or more to 10,000 U per 1 g of reaction liquid, and can further be added in 5 U or more to 1,000 U.

Furthermore, in the case when an immobilized enzyme is used, the immobilized enzyme can be added so as to have a mass including the mass of the support of 0.1% by mass or more and 200% by mass or less, further 1% by mass or more and 35% by mass or less (preferably 1% by mass or more and 20% by mass or less) with respect to the mass of the reaction liquid.

1 U for an enzyme activity refers to, in the case of a lipase, an amount of an enzyme that releases 1 $\mu$mol of a fatty acid in one minute in the hydrolysis of olive oil.

<Water Content>

In the production method of the present invention, by setting the water content in the reaction liquid of the above-mentioned esterification reaction to 0.4% by mass or more, the stability of the lipase is increased, and thus can be repetitively used. Furthermore, by introducing the glycerin generated by the esterification reaction into water, the solidification of the glycerin in the oil can be prevented, and thus the esterification reaction can be progressed smoothly.

Since the stability of the lipase can further be increased, the above-mentioned water content is further preferably 0.5% by mass or more and 20% by mass or less, and 0.5% by mass or more and 10% by mass or less.

When the water content is lower than 0.4% by mass, the stability of the lipase is lowered, and thus the esterification reaction is not progressed.

Furthermore, when the water content is 20% by mass or less, the contact of the DHA-containing triglyceride with the lipase tends to increase, and the content of the DHA-containing glyceride in the obtained composition containing a DHA-containing glyceride tends to increase. Furthermore, the hydrolysis reaction is difficult to progress, and thus the acid value tends to be kept low.

The water may be sequentially added, may be continuously added, or may be added in a lamp to the reaction liquid.

<Acid Value>

In the present invention, the acid value refers to an acid value of an oil content extracted from a sample (the standard methods for the analysis of fats, oils and related materials established by the Japan Oil Chemist's Society (Standard methods for the analysis of fats, oils and related materials, edited by the Specification Test Committee of the Japan Oil Chemist's Society): established by the Japan Oil Chemist's Society, 2013 1.5 Acid Value of Extract Oil)).

Since a composition containing a DHA-containing glyceride having high purity and fine quality can be obtained by the esterification reaction, it is preferable that the above-mentioned composition containing a DHA-containing glyceride has an acid value of 12 or less, further 2.2 or more and 12 or less, 3 or more and 9 or less.

That the above-mentioned acid value is higher than 12 means that the concentration of the free fatty acid is high. Therefore, when the acid value is higher than 12, oxidation deterioration of the composition containing a DHA-containing glyceride occurs, and thus a composition containing a DHA-containing glyceride having fine quality cannot be obtained.

Furthermore, since it is difficult to separate the free fatty acid and glyceride in the purification step, the burden in the production process is much, and thus a DHA-containing glyceride cannot be efficiently obtained.

In the present invention, the free fatty acid refers to a fatty acid that is not present as a fatty acid esterified product (a non-ester-bond type fatty acid).

<Method for Measuring Acid Value>

In the present invention, the acid value can be measured by the standard methods for the analysis of fats, oils and related materials established by the Japan Oil Chemist's Society (the Standard methods for the analysis of fats, oils and related materials, edited by the Specification Test Committee of the Japan Oil Chemist's Society): established by the Japan Oil Chemist's Society, 2013 1.5 Acid Value of Extract Oil).

Specifically, firstly, a sample (a reaction liquid after completion of a reaction) was correctly weighed in an Erlenmeyer flask according to a collection amount corresponding to a presumed acid value thereof, 100 mL of a mixed solvent of ethanol/diethyl ether = 1/1 (w/w) was added, and the sample was completely dissolved. Secondly, the solution was titrated with 0.1 mol/L of ethanolic potassium hydroxide, and the point at which the color change of a phenol phthalein solution added as an indicator had continued for 30 seconds or more was deemed as an end point. The acid value was calculated by the following formula (3).

$$\text{Acid Value} = 5.611 \times A \times F/B \ \ldots (3)$$

A: Use amount of 0.1 mol/L ethanolic potassium hydroxide (mL)
B: Amount of collected sample (g)
F: Factor of ethanolic potassium hydroxide

<Lower Alcohol>

The lower alcohol used in the production method of the present invention refers to a monoalcohol having 1, 2 or 3 carbon atom(s). Specifically, the lower alcohol is preferably methanol and/or ethanol, further preferably ethanol, in that it is excellent in miscibility with water.

<Mass of lower alcohol with respect to raw material oil and fat>

It is preferable that the above-mentioned reaction liquid contains 0.1 parts by mass or more and 3.0 parts by mass or less, further 0.3 parts by mass or more and 2.5 parts by mass of a lower alcohol with respect to 9.5 parts by mass of the above-mentioned raw material oil and fat, since the esterification reaction can be smoothly progressed by mixing the reaction liquid with both the water and the oil and fat.

The lower alcohol may be continuously added, may be added stepwise, or may be added in a lump to the above-mentioned reaction liquid, and it is preferable to add continuously or in a lump since the deactivation of the lipase can be suppressed.

In the present invention, "the lower alcohol is continuously added", refers to that the lower alcohol is continued to be added, whereas "the lower alcohol is added stepwise" refers to that the lower alcohol is added in plural times, but not in a continuous manner.

<Mass of lower alcohol with respect to water>

The mass ratio of the lower alcohol with respect to the water in the above-mentioned reaction liquid (lower alcohol /water) can be 1 or more and 50 or less, and can further be 2 or more and 20 or less, since the deactivation of the lipase can be suppressed.

<Esterification Reaction>

<Esterification rate>

In the above-mentioned esterification reaction, since the mass ratio of the DHA with respect to the EPA in the glycerides in the composition containing a DHA-containing glyceride (DHA / EPA) can be increased, the rate of the lower alcohol fatty acid esterified product with respect to the oil and fat components in the composition containing a DHA-containing glyceride (hereinafter also referred to as "esterification rate") may be 40% by mass or more, and may further be 50% by mass or more and 90% by mass or less, 60% by mass or more and 80% by mass or less.
The rate of the oil and fat components contained in the above-mentioned composition containing a DHA-containing glyceride can be 80% by mass or more and 100% by mass or less, and can further be 90% by mass or more and 100% by mass or less.

<Reaction Temperature and Reaction Time>

The temperature of the reaction liquid in the above-mentioned esterification reaction may be appropriately determined depending on the kind of the lipase to be used, and specifically, the temperature can be, for example, 15°C or more and 50°C or less, and can further be 25°C or more and 45°C or less.
Furthermore, the reaction time can be 2 hours or more and 48 hours or less, and can further be 4 hours or more and 36 hours or less.

<Reaction Pathway>

In the esterification reaction, generally, an alcohol and a glyceride are reacted under a condition of a small water content. The reason therefor is that, when the water content is high, the ester bond of the generated fatty acid esterified product may be hydrolyzed, or a free fatty acid may be generated.
By contrast, in the production method of the present invention, even when the water content in the reaction liquid is 0.4% by mass or more, a composition containing a DHA-containing glyceride having an acid value of 12 or less, having high purity and fine quality can be efficiently produced.
Furthermore, the stability of the lipase can be increased by the water content contained in the reaction liquid, and consequently, repetitive utilization of the lipase is enabled.

[Molecular Distillation]

The production method of the present invention can further include a step of subjecting the composition containing a DHA-containing glyceride obtained in the esterification reaction (Step 1 of FIG. 1) to molecular distillation (Step 2 of FIG. 1).
Molecular distillation is distillation conducted under a high vacuum degree. By the molecular distillation step, the composition containing a DHA-containing glyceride can be separated into glycerides and other components, and consequently, a composition containing a DHA-containing glyceride having a glyceride content of 70% by mass or more and 100% by mass or less can be obtained.

<Distillation Temperature and Vacuum Degree>

The temperature in the molecular distillation can be 80°C or more and 200°C or less, and can further be 150°C or more and 200°C or less. Furthermore, the vacuum degree can be 0.001 Torr or more and 5 Torr or less, and can further be 0.01 Torr or more and 1 Torr or less.
Specifically, it is preferable that the temperature is 140°C or more and 160°C or less, and the vacuum degree is 0.01 Torr or more and 0.1 Torr or less.
As the apparatus used for the molecular distillation, the molecular distillation can be generally conducted by using a commercially available apparatus. Specifically, for example, a centrifuge molecular distillator, a short pass distillator, a dropping membrane distillator and the like can be used, and it is specifically preferable to use a short pass distillator.

<Hydrolysis Reaction>

The production method of the present invention can further include a step of subjecting the composition containing a DHA-containing glyceride (second composition) obtained by conducting the esterification reaction (Step 1 of FIG. 1) and then conducting the molecular distillation (Step 2 of FIG. 1) to a hydrolysis reaction and subjecting the product to a molecular distillation treatment (Step 3 of FIG. 1).

By reacting the lipase with the above-mentioned composition containing a DHA-containing glyceride (second composition) in the presence of water to conduct a hydrolysis reaction, and separating the glyceride and the other components by the molecular distillation treatment, a composition containing a DHA-containing glyceride having high purity can be obtained even from a raw material oil and fat having a low DHA content such as sardine oil (DHA content: 12% by mass). Specifically, a composition containing a DHA-containing glyceride having a proportion of the DHA contained as a constituent fatty acid in the glycerides of the above-mentioned glyceride-containing composition of 30% by mass or more and 80% by mass or less can be obtained.

<Water Content against Raw Material Oil and Fat>

In the above-mentioned hydrolysis reaction, since the mass ratio of the DHA with respect to the EPA in the glycerides in the composition containing a DHA-containing glyceride (DHA / EPA), and the proportion of the DHA contained in the glyceride as a constituent fatty acid can be increased, the water content with respect to the raw material oil and fat is preferably 10% by mass or more and 100% by mass or less, further preferably 10% by mass or more and 50% by mass or less, and even more preferably 20% by mass or more and 40% by mass or less.

<Reaction Temperature and Reaction Time>

The temperature of the reaction liquid in the above-mentioned hydrolysis reaction may be appropriately determined depending on the kind of the used lipase, and specifically, the temperature can be, for example, 15°C or more and 50°C or less, further 20°C or more and 45°C or less.

Furthermore, the reaction time can be 5 hours or more and 48 hours or less, and can further be 10 hours or more and 24 hours or less.

<Lipase>

The lipase used in the above-mentioned hydrolysis reaction is not specifically limited as long as it catalyzes the hydrolysis reaction and has a property that it is difficult to react with highly unsaturated fatty acids.

Examples include lipases obtained from microorganisms belonging to Candida cylindoracea (lipase OF: Meito Sangyo Co., Ltd.), lipases obtained from microorganisms belonging to Alcaligenes sp. (lipase QLM, lipase QLC and lipase PL: all of these are manufactured by Meito Sangyo Co., Ltd.), lipases obtained from microorganisms belonging to Burkholderia cepacia (lipase PS: manufactured by Amano Enzyme, Inc.), lipases obtained from microorganisms belonging to Pseudomonas fluorescens (lipase AK: manufactured by Amano Enzyme, Inc.), lipases obtained from microorganisms belonging to Thermomyces lanuginosa) (LypoZyme TLIM: manufactured by Novozyme), and the like.

<Use Amount of Lipase>

The use amount of the lipase is not specifically limited as long as the hydrolysis reaction progresses, and for example, in the case when an enzyme having a free form is used, the enzyme can be generally added in 1 unit (U) or more and 10,000 U per 1 g of the reaction liquid addition, and can further be added in 5 U or more and 5,000 U.

<Action and Effect>

Before explaining the action and effect of the production method of the present invention, firstly, a known method for producing a composition containing a DHA-containing glyceride will be explained.

<Known Method for Producing Composition Containing DHA-Containing Glyceride (Patent Literature 1)>

In the known method for producing a composition containing a DHA-containing glyceride (described in Patent Literature 1), firstly, a hydrolysis reaction is conducted by reacting an enzyme dissolved in purified water with an oil and fat containing highly unsaturated fatty acids as constituent fatty acids to prepare a mixture containing a monoglyceride and free fatty acids. Furthermore, the above-mentioned mixture is fractionated by centrifugation liquid-liquid partition chromatography, whereby a fraction containing free fatty acids of highly unsaturated fatty acids including DHA and a monoglyceride can be obtained.

<Known Method for Producing Composition Containing DHA-Containing Glyceride (Patent Literature 1) - Problem>

However, a monoglyceride fraction obtained by the method described in Patent Literature 1 contains other unsaturated fatty acids such as EPA besides DHA, and thus DHA cannot be selectively concentrated. In order to separate a desired DHA-containing glyceride from such monoglyceride fraction containing plural kinds of highly unsaturated fatty acids, for example, it is necessary to conduct distillation in which the conditions (for example, vacuum degree, heating temperature, heating method and heating time) are very strictly controlled, and thus the separation is difficult.
Furthermore, since large amounts of free fatty acids are generated in the above-mentioned production method, a DHA-containing monoglyceride having high purity cannot be obtained.
In addition, a DHA-containing diglyceride and a DHA-containing triglyceride cannot be obtained by the above-mentioned production method.

<Known Method for producing Composition Containing DHA-Containing Glyceride (Patent Literature 2)>

The known method for producing a composition containing a DHA-containing glyceride (described in Patent Literature 2) describes a method for obtaining a glyceride fraction containing concentrated DHA, comprising: Step a, in which a lipase is firstly reacted with a raw material oil and fat, and a distillation treatment is further conducted, whereby a glyceride fraction containing DHA and EPA is separated; and Step b, in which a lipase is then reacted with the above-mentioned glyceride fraction to give a glyceride fraction containing concentrated DHA.

<Known Method for producing Composition Containing DHA-Containing Glyceride (Patent Literature 2) - Problem>

However, in the production method described in Patent Literature 2, it is necessary to undergo the two enzyme reaction steps: Steps a and b. Therefore, the loss of the intended product in the production steps is much, and thus a glyceride fraction in which a sufficient amount of DHA is concentrated cannot be obtained.
Furthermore, since free fatty acids are generated in large amounts in the above-mentioned production method and the acid value increases, the quality of the composition containing a DHA-containing glyceride is lowered.

<Action and Effect of Production Method of Present Invention>

By contrast, according to the production method of the present invention, a composition containing a DHA-containing glyceride having a low acid value can be efficiently obtained, by conducting an esterification reaction by reacting a lipase with a raw material oil and fat containing highly unsaturated fatty acids as constituent fatty acids in the presence of water and a lower alcohol to thereby control the water content in the reaction liquid of the above-mentioned reaction.

(i) Specifically, by adjusting the acid value of the above-mentioned composition containing a DHA-containing glyceride to 12 or less, a composition containing a DHA-containing glyceride having fine quality with small free fatty acids can be obtained.
Furthermore, by decreasing the content of the free fatty acid, the separation of the DHA-containing glyceride can made easy, and thus the yield of the intended product can be increased.
In a general esterification reaction, since a hydrolysis reaction easily progresses in the presence of water and thus free fatty acids are generated, an esterification reaction is generally conducted under a condition in which water content is small or water is absent. However, in the production method of the present invention, the acid value can be kept low even at a water content of 0.4% by mass or more.
Furthermore, the DHA content in the glycerides of the composition containing a DHA-containing glyceride can be increased, and a composition containing a DHA-containing glyceride having high purity can be obtained.
(ii) According to the production method of the present invention, the mass ratio of the DHA with respect to the EPA in the glycerides of the composition containing a DHA-containing glyceride can be increased. Since EPA and DHA have similar molecular weights and similar numbers of double bonds, it is difficult to isolate only one of the DHA-containing glycerides. Therefore, by increasing the rate of the DHA with respect to the EPA in the composition containing a DHA-containing glyceride, the loss of the intended product in the purification step can be decreased, and thus a composition containing a DHA-containing glyceride can be obtained more efficiently.

Furthermore, since the total content of the monoglyceride, diglyceride and triglyceride in the composition containing a DHA-containing glyceride, and the contents of the respective glycerides can be increased, the yield of the composition containing a DHA-containing glyceride can be increased.

<DHA-Containing Glyceride-Containing Composition>

A composition containing a DHA-containing glyceride that can be obtained by the above-mentioned esterification reaction is referred to as a first composition.

A composition containing a DHA-containing glyceride that can be obtained by the molecular distillation treatment subsequent to the above-mentioned esterification reaction is referred to as a second composition.

Furthermore, a composition containing a DHA-containing glyceride that can be obtained by the molecular distillation treatment after the hydrolysis reaction subsequent to the above-mentioned molecular distillation treatment is referred to as a third composition.

1. First Composition

The first composition obtained in the present invention is a composition containing a DHA-containing glyceride having an acid value of 12 or less,
having a glyceride content of 20% by mass or more and 60% by mass or less with respect to the oil and fat components in the above-mentioned composition, and containing DHA and EPA as constituent fatty acids at the following mass ratio in the above-mentioned glyceride:

$$0.5 \leq (DHA\,/\,EPA \text{ in glycerides}) \leq 10.$$

The above-mentioned glyceride content is further preferably 25% by mass or more and 55% by mass or less, 30% by mass or more and 50% by mass or less.

The above-mentioned acid value is further preferably 2.2 or more and 12 or less, 3 or more and 9 or less.

It is further preferable that the mass ratio of the DHA with respect to the above-mentioned EPA is the following ratio:

$$1 \leq (DHA\,/\,EPA \text{ in glycerides}) \leq 5.$$

<DHA Content Contained as Constituent Fatty Acid>

In the above-mentioned first composition, the proportion of the DHA contained as a constituent fatty acid in the glycerides is preferably 20% by mass or more and 90% by mass or less, further preferably 20% by mass or more and 70% by mass or less, 25% by mass or more and 60% by mass or less.

<Proportion of Monoglyceride and Diglyceride>

The proportion of the monoglyceride with respect to the oil and fat components in the above-mentioned first composition is preferably 1% by mass or more and 55% by mass or less, and the proportion of the diglyceride is preferably 1% by mass or more and 35% by mass or less.

The proportion of the monoglyceride is preferably 5% by mass or more and 50% by mass or less, and the proportion of the diglyceride is preferably 5% by mass or more and 30% by mass or less.

In addition, the proportion of the triglyceride can be 1 mass or more and 30% by mass or less, and can further be 1.5% by mass or more and 20% by mass or less.

2. Second Composition

The second composition obtained in the present invention is a composition containing a DHA-containing glyceride having an acid value of 12 or less, having a glyceride content of 70% by mass or more and 100% by mass or less with respect to the oil and fat components in the above-mentioned composition, and containing DHA and EPA as constituent fatty acids in the above-mentioned glyceride at the following mass ratio:

$$0.5 \le (\text{DHA} / \text{EPA in glycerides}) \le 10.$$

The above-mentioned glyceride content is further preferably 80% by mass or more and 100% by mass or less, 90% by mass or more and 100% by mass or less.

The above-mentioned acid value is further preferably 0 or more and 12 or less (2.2 or more and 12 or less), 0.2 or more and 9 or less (for example, 3 or more and 9 or less).

The mass ratio of the DHA with respect to the above-mentioned EPA is further preferably the following ratio:

$$1 \le (\text{DHA} / \text{EPA in glycerides}) \le 5.$$

<DHA Content Contained as Constituent Fatty Acid>

In the above-mentioned second composition, the proportion of the DHA contained as a constituent fatty acid in the glycerides is preferably 20% by mass or more and 90% by mass or less, further preferably 20% by mass or more and 70% by mass or less, 25% by mass or more and 60% by mass or less.

<Proportion of Monoglyceride and Diglyceride>

It is preferable that the proportion of the monoglyceride with respect to the oil and fat components in the above-mentioned second composition is 5% by mass or more and 45% by mass or less, and the proportion of the diglyceride is 30% by mass or more and 90% by mass or less.

Furthermore, the proportion of the monoglyceride is preferably 10% by mass or more and 40% by mass or less, and the proportion of the diglyceride is preferably 40% by mass or more and 80% by mass or less.

The proportion of the triglyceride can be 1 mass or more and 25% by mass or less, and can further be 2% by mass or more and 20% by mass or less.

<DHA Content Contained in Each Glyceride Fraction>

In the above-mentioned second composition, the content of DHA contained in the monoglyceride can be 15% by mass or more and 50% by mass or less, and can further be 20% by mass or more and 40% by mass or less.

Furthermore, the content of the DHA contained in the diglyceride can be 20% by mass or more, and can further be 25% by mass or more and 45% by mass or less.

Furthermore, the content of the DHA contained in the triglyceride can be 10% by mass or more and 45% by mass or less, and can further be 15% by mass or more and 35% by mass or less.

3. Third Composition

The third composition obtained in the present invention is a composition containing a DHA-containing glyceride containing the DHA contained as a constituent fatty acid in the glycerides at a proportion of 30% by mass or more and 80% by mass or less.

The proportion of the above-mentioned DHA may further be 35% by mass or more and 75% by mass or less, or 40% by mass or more and 70% by mass or less.

<Acid Value>

The above-mentioned third composition has an acid value of preferably 0.1 or more and 5 or less, further preferably 0.1 or more and 3 or less.

<Glyceride Content>

The glyceride content with respect to the oil and fat components in the above-mentioned third composition is preferably 80% by mass or more and 100% by mass or less, and is further preferably 85% by mass or more and 100% by mass or less, 90% by mass or more and 100% by mass or less.

<Mass Ratio of DHA with respect to EPA in glyceride>

A composition containing a DHA-containing glyceride contains DHA and EPA as constituent fatty acids in the glycerides of the third composition at the following mass ratio:

$$2 \le (DHA / EPA \ in \ glycerides) \le 15.$$

The mass ratio of the DHA with respect to the above-mentioned EPA is further preferably the following ratio:

$$2.5 \le (DHA / EPA \ in \ glycerides) \le 10$$

$$2.5 \le (DHA / EPA \ in \ glycerides) \le 6.$$

<Proportion of Monoglyceride>

The proportion of the monoglyceride with respect to the oil and fat components in the above-mentioned third composition is preferably 0% by mass or more and 20% by mass or less, further preferably 0% by mass or more and 15% by mass or less, 0% by mass or more and 10% by mass or less.

<Proportion of Diglyceride>

The proportion of the diglyceride with respect to the oil and fat components in the above-mentioned third composition is preferably 5% by mass or more and 50% by mass or less, and further preferably 10% by mass or more and 45% by mass or less, 15% by mass or more and 40% by mass or less.

<Proportion of Triglyceride>

The proportion of the triglyceride with respect to the oil and fat components in the above-mentioned third composition is preferably 40 mass or more and 95% by mass or less, and further preferably 45% by mass or more and 95% by mass or less, 50% by mass or more and 90% by mass or less.

<Specific Examples of Composition Containing DHA-Containing Glyceride >

The composition containing a DHA-containing glyceride (the first to third compositions) can be used as raw materials for pharmaceuticals, cosmetic preparations, foods or forages. Examples include DHA-containing monoglycerides, DHA-containing diglycerides or DHA-containing triglycerides. The composition containing a DHA-containing glyceride can be used as raw materials for food compositions such as supplements and capsule formulations.

**Examples**

Next, the present invention will further be explained based on Examples and Comparative Examples. However, the present invention is not limited to these Examples.

[Preparation Example 1 (preparation of immobilized enzyme)]

A solution of 400 g of Thermomyces lanuginosus (940 KLU/mL) was sprayed onto 1 kg of celite 545 (Johns-Manville Corporation, particle size: 0.02-0.1 mm) by using a fluidized bed granulator manufactured by Okawara Corporation.

The above-mentioned lipase solution was fed through a Perista pump (Tokyo Rikakikai Co., Ltd.). The temperature of the air at the inlet by an airflow at 100 m$^3$/h was 57°C, and the temperature of the immobilized product was about 40°C.

After the immobilization was completed, the lipase solution was further dried in the fluidized bed for 5 minutes to give an particulate immobilized enzyme (average particle diameter: 600 μm, specific gravity: 2). Thermomyces lanuginosus is a 1,3-specific lipase, and the method for immobilizing on the support is a physical adsorption process.

[Preparation Example 2 (preparation of immobilized enzyme)]

70% by mass of divinylbenzene (DVB), 15% by mass of glycidyl methacrylate and 15% by mass of DEAE methacrylate were copolymerized by a general method to give a particulate resin support. This resin support had an average fine pore diameter of 11.5 nm, a fine pore volume of 0.5 cm$^3$/g, an average particle diameter of 0.5 mm, and a specific gravity of 0.2. 10 L of a 2% by mass aqueous solution of a lipase derived from Rhizopus sp. FAP-15 (manufactured by Amano Enzyme, Inc. 155,000 u/g) was added to 1 kg of the obtained resin support, and immobilized at 25°C for 3 hours under stirring.

The resin support was filtered and washed, then dried in a vacuum drier for 2 hours to give an immobilized enzyme. Lipase FAP-15 is a 1,3-specific lipase, and the method for immobilizing on the support is a covalent bonding process.

[Example 1 (esterification reaction)]

One kilogram of a purified fish oil (sardine oil: acid value: 0, triglyceride content: 95% by mass, DHA content in fatty acid composition 12% by mass, EPA/DHA in fatty acid composition (mass ratio: 0.5 or more and 6 or less) was put into a separable flask (volume: 3 L), and 52.5 g of ethanol was added.

The flask was mixed to allow the ethanol to homogeneously disperse in the fish oil. Secondly, 5.25 g of water (water content in reaction liquid: 0.5% by mass) was put into the flask, the mixture was stirred to allow the water to disperse in the fish oil-ethanol mixture, whereby a reaction liquid was prepared.

Subsequently, 105 g of the immobilized enzyme prepared in Preparation Example 1 was added, the air in the sample bottle was purged with nitrogen, and the sample was then reacted by using a stirrer at 150 rpm and 30°C for 24 hours to give a composition containing a DHA-containing glyceride (first composition).

200 μL of the reaction liquid was collected at each of the timepoints of 0 hour, 2 hours, 4 hours, 6 hours and 24 hours from the initiation of the reaction, and subjected to component analysis. Specifically, the analysis is the quantification of the esterification rate (% by mass), the contents of the triglyceride, diglyceride and monoglyceride (% by mass), the glyceride content (% by mass), the contents of DHA and EPA in the glycerides (% by mass), and the mass ratio of the DHA with respect to the EPA in the glycerides (DHA / EPA).

Furthermore, 52.5 g of ethanol was added to the reaction liquid at the timepoints of 2, 4 and 6 hours from the initiation of the reaction, and nitrogen purging was conducted in the sample bottle. Furthermore, a reaction for 24 hours was set as one cycle, and the reaction was repetitively conducted 3 cycles by recycling the immobilized enzyme used in the above-mentioned reaction.

After the completion of each cycle, the oil and the immobilized enzyme were separated from the reaction liquid by means of aspiration filtration, the separated immobilized enzyme was transferred to the reaction container, and necessary amounts of oil, water and ethanol were added thereto, and the mixture was repetitively used in the reaction of the next cycle.

Furthermore, a minor amount of the reaction liquid was collected at each of the timepoints of 0, 2, 4, 6, 8 and 24 hours (the time when the reaction had completed) from the initiation of the reaction, and subjected to component analysis.

[Examples 2 to 6 (esterification reaction)]

The composition containing a DHA-containing glycerides of Examples 2 to 6 were obtained by a similar method to that of Example 1, except that the amount of the used water was changed to 10.5 g (water content in reaction liquid: 1% by mass), 21 g (water content in reaction liquid: 2% by mass), 52.5 g (water content in reaction liquid: 5% by mass), 105 g (water content in reaction liquid: 9% by mass), and 210 g (water content in reaction liquid: 17% by mass).

[Comparative Examples 1 to 4 (esterification reaction)]

The composition containing a DHA-containing glycerides of Comparative Examples 1 to 4 were obtained by a similar method to that of Example 1, except that the amount of the used water was changed to 0 g (water content in reaction liquid: 0%), 3.15 g (water content in reaction liquid: 0.3% by mass), 525 g (water content in reaction liquid: 33% by mass) and 1050 g (water content in reaction liquid: 50% by mass).

In Examples 1 to 6 and Comparative Examples 1 to 4, the component analysis value was a value measured by the TLC-FID or GC analysis mentioned below, and the acid value is a value measured by the above-mentioned method. Furthermore, the esterification rate was calculated by the following formula.

$$\text{Esterification rate (\% by mass)} = (\text{peak surface area of fatty acid}$$

$$\text{ethyl ester / total peak surface area of sample}) \times 100$$

1) Lipid Composition Analysis (TLC-FID)

The sample (reaction liquid) after the esterification reaction was centrifuged at 8,000 rpm for 5 minutes to remove the aqueous phase, and a 1% hexane solution of the obtained oil phase was prepared and subjected to TLC-FID analysis using Iatroscan MK-6 (manufactured by Mitsubishi Chemical Medience). Primary development was conducted by about 60% by using toluene: chloroform: acetic acid (50 : 30 : 0.75, v/v/v) as solvents, and secondary development was conducted by 100% by using hexane: diethyl ether (65: 5, v/v) as solvents. By said lipid composition analysis, the lipid compositions existing in the reaction liquid (more specifically, the contents of the fatty acid ethyl ester, triglyceride, diglyceride and monoglyceride) can be identified. By this way, the above-mentioned esterification rate can be calculated.

2) Thin layer TLC

100 $\mu$L was collected from the sample after the esterification reaction (reaction liquid), the glyceride fractions other than the ethyl ester were scraped off by preparation TLC, the above-mentioned glyceride fraction was methyl-esterified, and the fatty acid composition was analyzed by GC analysis. Specifically, a 5% hexane solution of the reaction liquid was prepared, applied onto a preparation TLC and developed with chloroform : acetone : acetic acid = 90: 10: 0.5. After the development, the respective glyceride fractions were scraped off, directly methyl-esterified, and analyzed by GC analysis under the analysis conditions mentioned below.

3) GC (gas chromatography analysis)

Column: Omegawax 250 manufactured by Supelco (L $\times$ I. D. 30 m $\times$ 0.25 mm, df: 0.25 $\mu$m)
Carrier gas: helium, column flow amount: 1 mL/min
Detector: FID, 250°C (hydrogen: about 30 mL/min, air: about 300 mL/min, nitrogen: about 18 mL/min)
Injection port: 250°C, split ratio (60: 1)
Column oven temperature: 50°C, 2 min; 4°C/min, 220°C (15 min)
Apparatus: 6890 N manufactured by Agilent Technologies
Temperature rising condition: oven: 205°C, retention for 10 minutes $\rightarrow$ temperature rising: 2.5°C/min $\rightarrow$ 240°C, retention for 11 minutes
Analysis time: 35 min

By said GC analysis, the contents of the DHA and EPA in the glycerides of the composition containing a DHA-containing glyceride (% by mass), and the DHA / EPA in the glycerides (mass ratio) can be identified.

The results of the component analyses of the composition containing a DHA-containing glycerides obtained in Examples 1 to 6 and Comparative Examples 1 to 4 are shown in Table 1. In addition, the acid values of the composition containing a DHA-containing glycerides obtained in Examples 1 to 6 were 12 or less.

[Table 1]

| Example 1 (Water content: 0.5% by mass) | | | | | | | | | | |
|---|---|---|---|---|---|---|---|---|---|---|
| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
| 1 cycle | 2 | 19.5 | 21.9 | 47.4 | 11.2 | 102.0 | 18.7 | 14.5 | 0.8 | |
| | 4 | 34.0 | 10.2 | 36.2 | 19.8 | 66.1 | 19.1 | 16.9 | 0.9 | |
| | 6 | 42.8 | 6.7 | 28.0 | 21.5 | 56.2 | 18.9 | 19.3 | 1.0 | |
| | 8 | 46.4 | 5.1 | 23.6 | 23.8 | 52.5 | 18.3 | 20.3 | 1.1 | |
| | 24 | 58.9 | 1.7 | 14.1 | 25.2 | 40.9 | 16.8 | 24.2 | 1.4 | 3.2 |
| 2 cycles | 2 | 17.7 | 29.4 | 32.4 | 20.6 | 82.4 | 18.3 | 14.1 | 0.8 | |
| | 4 | 35.4 | 15.5 | 26.3 | 21.6 | 63.4 | 18.8 | 17.4 | 0.9 | |
| | 6 | 36.1 | 7.8 | 25.8 | 30.4 | 63.9 | 18.6 | 17.4 | 0.9 | |
| | 8 | 33.2 | 5.1 | 22.3 | 39.4 | 66.7 | 18.3 | 16.8 | 0.9 | |
| | 24 | 39.9 | 0.9 | 14.0 | 45.3 | 60.1 | 17.3 | 18.0 | 1.0 | 2.6 |
| 3 cycles | 2 | 20.6 | 40.4 | 25.3 | 13.7 | 79.4 | 18.6 | 14.5 | 0.8 | |
| | 4 | 25.4 | 16.9 | 32.1 | 25.6 | 74.6 | 18.6 | 15.3 | 0.8 | |
| | 6 | 30.5 | 9.5 | 28.7 | 31.4 | 69.6 | 18.6 | 16.3 | 0.9 | |
| | 8 | 32.0 | 5.5 | 25.2 | 37.5 | 68.1 | 18.5 | 16.6 | 0.9 | |
| | 24 | 56.3 | 1.3 | 12.4 | 30.1 | 43.7 | 16.4 | 23.7 | 1.4 | 2.8 |

**Example 2 (Water content: 1% by mass)**

|  | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 1 cycle | 2 | 18.5 | 28.5 | 37.0 | 15.3 | 80.8 | 18.0 | 14.3 | 0.8 | |
| | 4 | 31.2 | 16.4 | 28.7 | 23.7 | 68.8 | 17.9 | 16.4 | 0.9 | |
| | 6 | 41.4 | 10.1 | 19.1 | 29.4 | 58.6 | 17.5 | 18.6 | 1.1 | |
| | 8 | 43.1 | 8.5 | 17.9 | 30.5 | 56.9 | 16.3 | 18.6 | 1.1 | |
| | 24 | 65.7 | 4.4 | 8.6 | 21.3 | 34.3 | 13.2 | 25.8 | 2.0 | 4.6 |
| 2 cycles | 2 | 12.9 | 60.2 | 15.5 | 11.4 | 87.1 | 18.0 | 13.2 | 0.7 | |
| | 4 | 24.0 | 39.5 | 18.2 | 18.3 | 76.0 | 17.9 | 14.7 | 0.8 | |
| | 6 | 25.7 | 31.8 | 18.5 | 23.9 | 74.2 | 17.6 | 14.9 | 0.8 | |
| | 8 | 33.4 | 19.6 | 14.4 | 32.6 | 66.6 | 16.7 | 16.2 | 1.0 | |
| | 24 | 51.8 | 4.4 | 13.0 | 30.2 | 47.6 | 15.1 | 20.4 | 1.4 | 6.0 |
| 3 cycles | 2 | 14.5 | 51.2 | 16.8 | 17.5 | 85.5 | 18.2 | 13.6 | 0.7 | |
| | 4 | 25.1 | 24.6 | 20.3 | 30.1 | 75.0 | 18.1 | 15.1 | 0.8 | |
| | 6 | 33.4 | 14.8 | 19.4 | 30.3 | 64.5 | 17.6 | 16.8 | 1.0 | |
| | 8 | 49.8 | 10.8 | 15.4 | 24.0 | 50.2 | 16.1 | 21.1 | 1.3 | 6.2 |

**Example 3 (Water content: 2% by mass)**

|  | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 1 cycle | 2 | 15.6 | 33.7 | 37.6 | 13.2 | 84.5 | 17.9 | 13.8 | 0.8 | |
| | 4 | 32.8 | 14.9 | 28.0 | 24.3 | 67.2 | 17.9 | 16.7 | 0.9 | |
| | 6 | 40.8 | 13.6 | 22.2 | 23.4 | 59.2 | 17.5 | 18.3 | 1.0 | |
| | 8 | 50.9 | 11.4 | 16.3 | 21.4 | 49.1 | 15.4 | 20.8 | 1.4 | |
| | 24 | 69.9 | 4.2 | 8.2 | 17.6 | 30.0 | 12.2 | 26.9 | 2.2 | 6.0 |

EP 3 276 007 A1

Example 3 (Water content: 2% by mass)

| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 2 cycles | 2 | 25.9 | 26.4 | 28.8 | 18.9 | 74.1 | 18.2 | 15.1 | 0.8 | |
| | 4 | 37.6 | 11.0 | 24.5 | 26.9 | 62.4 | 17.5 | 17.2 | 1.0 | |
| | 6 | 44.9 | 6.6 | 17.7 | 30.7 | 55.0 | 16.8 | 18.9 | 1.1 | |
| | 8 | 49.5 | 6.5 | 13.4 | 30.5 | 50.4 | 15.4 | 20.1 | 1.3 | |
| | 24 | 67.5 | 2.0 | 7.0 | 23.4 | 32.4 | 12.0 | 25.1 | 2.1 | |
| 3 cycles | 2 | 23.0 | 29.7 | 28.1 | 19.1 | 76.9 | 18.2 | 14.8 | 0.8 | |
| | 4 | 37.8 | 11.3 | 22.8 | 27.4 | 61.5 | 17.4 | 17.5 | 1.0 | |
| | 6 | 47.3 | 6.8 | 17.2 | 27.3 | 51.3 | 16.4 | 20.0 | 1.2 | |
| | 8 | 55.1 | 3.9 | 11.7 | 29.3 | 44.9 | 14.7 | 22.2 | 1.5 | 6.2 |

Example 4 (Water content: 5% by mass)

| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | |
|---|---|---|---|---|---|---|---|---|---|---|
| 1 cycle | 2 | 16.5 | 29.0 | 40.0 | 14.4 | 83.4 | 17.9 | 13.9 | 0.8 | |
| | 4 | 34.8 | 14.0 | 27.6 | 23.6 | 65.2 | 17.9 | 17.2 | 1.0 | |
| | 6 | 42.9 | 9.8 | 20.1 | 27.2 | 57.1 | 17.2 | 18.9 | 1.1 | |
| | 8 | 52.2 | 7.7 | 14.1 | 26.0 | 47.8 | 15.4 | 21.3 | 1.4 | |
| | 24 | 70.6 | 2.0 | 7.2 | 18.1 | 27.3 | 11.0 | 26.7 | 2.4 | |
| 2 cycles | 2 | 31.2 | 16.8 | 31.0 | 21.0 | 68.8 | 17.9 | 16.0 | 0.9 | |
| | 4 | 41.4 | 11.5 | 24.5 | 22.6 | 58.6 | 16.7 | 18.1 | 1.1 | |
| | 6 | 57.1 | 5.5 | 13.0 | 24.4 | 42.9 | 15.1 | 23.2 | 1.5 | |
| | 8 | 55.4 | 4.8 | 10.1 | 29.6 | 44.5 | 14.1 | 21.7 | 1.5 | |
| | 24 | 73.0 | 2.4 | 5.4 | 18.5 | 26.3 | 10.2 | 28.1 | 2.7 | |

Example 4 (Water content: 5% by mass)

| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA |
|---|---|---|---|---|---|---|---|---|---|
| 3 cycles | 2 | 29.5 | 18.7 | 31.9 | 19.9 | 70.5 | 18.0 | 15.8 | 0.9 |
| | 4 | 50.3 | 8.7 | 19.2 | 21.6 | 49.5 | 16.3 | 20.8 | 1.3 |
| | 6 | 56.4 | 4.5 | 13.1 | 24.0 | 41.6 | 15.2 | 23.3 | 1.5 |
| | 8 | 60.0 | 3.5 | 10.5 | 26.0 | 40.0 | 13.4 | 23.9 | 1.8 |

Example 5 (Water content: 9% by mass)

| | Reaction time (hour) | Esterification rate (% by mass) | Triglyceride (% by mass) | Diglyceride (% by mass) | Mono glyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 1 cycle | 2 | 14.9 | 34.8 | 38.1 | 12.2 | 85.1 | 17.9 | 13.7 | 0.8 | |
| | 4 | 41.0 | 15.0 | 26.1 | 17.9 | 59.0 | 17.6 | 18.7 | 1.1 | |
| | 6 | 41.0 | 11.9 | 21.8 | 25.3 | 59.0 | 17.1 | 18.4 | 1.1 | |
| | 8 | 50.8 | 7.9 | 13.6 | 27.7 | 49.2 | 15.5 | 20.8 | 1.3 | |
| | 24 | 69.4 | 5.7 | 8.8 | 15.3 | 29.8 | 11.9 | 25.5 | 2.1 | 10.0 |
| 2 cycles | 2 | 22.4 | 25.5 | 32.2 | 19.2 | 76.9 | 18.4 | 14.7 | 0.8 | |
| | 4 | 33.7 | 15.2 | 27.2 | 24.0 | 66.4 | 17.7 | 16.6 | 0.9 | |
| | 6 | 47.9 | 8.0 | 17.2 | 26.9 | 52.1 | 16.5 | 20.0 | 1.2 | |
| | 8 | 46.6 | 8.7 | 15.4 | 29.3 | 53.4 | 15.5 | 19.2 | 1.2 | |
| | 24 | 75.5 | 3.7 | 6.4 | 14.8 | 24.9 | 9.5 | 30.2 | 3.2 | |
| 3 cycles | 2 | 18.1 | 35.8 | 30.6 | 15.6 | 82.0 | 18.3 | 14.0 | 0.8 | |
| | 4 | 33.0 | 16.3 | 25.4 | 25.2 | 66.9 | 17.8 | 16.4 | 0.9 | |
| | 6 | 42.3 | 9.5 | 20.7 | 26.4 | 56.6 | 16.7 | 18.4 | 1.1 | |
| | 8 | 56.3 | 4.2 | 13.0 | 25.3 | 42.5 | 15.0 | 22.9 | 1.5 | |

EP 3 276 007 A1

19

(continued)

| Example 6 (Water content: 17% by mass) | | | | | | | | | | |
|---|---|---|---|---|---|---|---|---|---|---|
| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
| 1 cycle | 2 | 16.2 | 38.9 | 29.3 | 11.3 | 79.5 | 17.6 | 14.1 | 0.8 | |
| 1 cycle | 6 | 50.2 | 12.5 | 21.0 | 15.6 | 49.1 | 16.6 | 21.8 | 1.3 | |
| 1 cycle | 8 | 60.4 | 6.8 | 14.4 | 18.4 | 39.6 | 15.9 | 25.9 | 1.6 | |
| 1 cycle | 24 | 74.9 | 7.3 | 0.6 | 13.5 | 21.4 | 13.1 | 35.1 | 2.7 | 11.0 |

| Comparative Example 1 (Water content: 0% by mass) | | | | | | | | | | |
|---|---|---|---|---|---|---|---|---|---|---|
| | Reaction time (hour) | Esterification rate (% by mass) | Triglyceride (% by mass) | Diglyceride (% by mass) | Mono glyceride (% by mass) | Glyceride content (% by mass) | EPA content in glycerides (% by mass) | DHA content in glycerides (% by mass) | DHA/ EPA | Acid value |
| 1 cycle | 2 | 12.0 | 28.1 | 39.7 | 19.3 | 87.1 | 18.8 | 13.5 | 0.7 | |
| 1 cycle | 4 | 17.3 | 16.5 | 36.5 | 29.7 | 82.7 | 19.2 | 14.3 | 0.7 | |
| 1 cycle | 6 | 22.6 | 10.3 | 29.9 | 36.6 | 76.8 | 19.4 | 15.2 | 0.8 | |
| 1 cycle | 8 | 22.3 | 9.0 | 30.6 | 38.2 | 77.8 | 19.1 | 15.1 | 0.8 | |
| 1 cycle | 24 | 32.1 | 4.9 | 21.6 | 41.4 | 67.9 | 18.4 | 17.0 | 0.9 | 1.5 |
| 2 cycles | 2 | 6.9 | 66.4 | 16.9 | 9.7 | 93.0 | 18.2 | 12.8 | 0.7 | |
| 2 cycles | 4 | 10.2 | 51.0 | 25.8 | 13.0 | 89.8 | 18.4 | 13.2 | 0.7 | |
| 2 cycles | 6 | 9.8 | 46.7 | 26.6 | 16.9 | 90.2 | 18.5 | 13.2 | 0.7 | |
| 2 cycles | 8 | 11.8 | 44.4 | 25.2 | 18.0 | 87.6 | 18.7 | 13.5 | 0.7 | |
| 2 cycles | 24 | 17.8 | 22.6 | 32.8 | 26.8 | 82.2 | 19.2 | 14.4 | 0.7 | |
| 3 cycles | 2 | 4.1 | 74.3 | 15.2 | 6.5 | 96.0 | 18.2 | 12.5 | 0.7 | |
| 3 cycles | 4 | 6.9 | 61.9 | 19.8 | 11.4 | 93.1 | 18.5 | 12.8 | 0.7 | |
| 3 cycles | 6 | 7.5 | 56.2 | 22.8 | 11.7 | 90.7 | 18.6 | 12.9 | 0.7 | |
| 3 cycles | 8 | 35.4 | 9.9 | 17.8 | 36.9 | 64.6 | 21.8 | 18.0 | 0.8 | |

(continued)

**Comparative Example 2 (Water content: 0.3% by mass)**

| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 1 cycle | 2 | 18.8 | 25.5 | 43.0 | 13.0 | 81.4 | 19.2 | 14.5 | 0.8 | |
| | 4 | 29.2 | 11.4 | 37.0 | 22.6 | 70.9 | 19.3 | 16.3 | 0.8 | |
| | 6 | 34.9 | 4.7 | 30.4 | 30.0 | 65.0 | 19.0 | 17.6 | 0.9 | |
| | 8 | 40.6 | 3.3 | 27.0 | 29.2 | 59.5 | 18.6 | 19.1 | 1.0 | |
| | 23 | 43.3 | 1.3 | 20.7 | 34.6 | 56.5 | 17.7 | 19.5 | 1.1 | 3.1 |
| 2 cycles | 2 | 15.0 | 37.7 | 31.0 | 16.5 | 85.1 | 18.6 | 13.7 | 0.7 | |
| | 4 | 20.6 | 18.9 | 35.8 | 24.0 | 78.7 | 19.1 | 14.6 | 0.8 | |
| | 6 | 24.3 | 15.5 | 34.8 | 25.6 | 75.9 | 19.4 | 15.3 | 0.8 | |
| | 8 | 22.4 | 10.8 | 35.7 | 31.0 | 77.5 | 19.2 | 15.0 | 0.8 | |
| | 23 | 24.1 | 3.5 | 28.1 | 44.5 | 76.0 | 19.1 | 15.3 | 0.8 | 2.6 |
| 3 cycles | 2 | 11.2 | 41.4 | 33.0 | 12.8 | 87.2 | 18.8 | 13.4 | 0.7 | |
| | 4 | 13.5 | 25.6 | 40.5 | 20.8 | 86.8 | 19.0 | 13.7 | 0.7 | |
| | 6 | 16.7 | 19.2 | 40.6 | 23.6 | 83.4 | 19.3 | 14.2 | 0.7 | |
| | 8 | 18.3 | 14.5 | 39.4 | 27.9 | 81.8 | 19.4 | 14.4 | 0.7 | |
| | 23 | 25.3 | 4.7 | 33.0 | 37.0 | 74.7 | 19.4 | 15.6 | 0.8 | 2.9 |

**Comparative Example 3 (Water content: 33% by mass)**

| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
|---|---|---|---|---|---|---|---|---|---|---|
| 1 cycle | 2 | 11.9 | 49.6 | 27.6 | 9.5 | 86.7 | 17.6 | 13.4 | 0.8 | |
| | 6 | 43.9 | 19.7 | 23.7 | 12.6 | 56.0 | 17.2 | 19.8 | 1.2 | |
| | 8 | 56.7 | 9.8 | 18.6 | 14.9 | 43.3 | 16.4 | 24.6 | 1.5 | |
| | 24 | 72.0 | 9.9 | 0.6 | 11.6 | 22.1 | 11.5 | 34.2 | 3.0 | 15.0 |

(continued)

| Comparative Example 4 (Water content: 50% by mass) | | | | | | | | | | |
|---|---|---|---|---|---|---|---|---|---|---|
| | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/ EPA | Acid value |
| 1 cycle | 2h | 8.6 | 49.8 | 30.8 | 10.1 | 90.7 | 17.7 | 13.0 | 0.7 | |
| | 6h | 34.1 | 28.6 | 26.6 | 9.6 | 64.8 | 16.9 | 17.3 | 1.0 | |
| | 8h | 38.7 | 17.7 | 22.8 | 20.3 | 60.8 | 17.2 | 18.4 | 1.1 | |
| | 24h | 64.4 | 16.8 | 0.7 | 12.8 | 30.3 | 10.1 | 26.5 | 2.6 | 17.0 |

22

It can be understood from Table 1 that a composition containing a DHA-containing glyceride (first composition) each having an acid value of 12 or less can be obtained by that the water content in the reaction liquid of the above-mentioned esterification reaction is 0.4% by mass or more (Examples 1 to 6).

<Regarding First Composition>

Furthermore, according to Examples 1 to 6, it can be understood that the obtained first compositions is a composition containing a DHA-containing glyceride each having an acid value of 12 or less, having a glyceride content of 20% by mass or more and 60% by mass or less with respect to the oil and fat components in the above-mentioned composition, and containing DHA and EPA as constituent fatty acids at the following mass ratio in the above-mentioned glycerides:

$$0.5 \leq (DHA / EPA \text{ in glycerides}) \leq 10.$$

It can be understood that compositions containing a DHA-containing glyceride each having a proportion of the DHA contained as a constituent fatty acid in the glycerides of the above-mentioned first composition of 20% by mass or more and 70% by mass or less are easily obtained.

It can be understood that compositions containing a DHA-containing glyceride each having a proportion of the monoglyceride of 1% by mass or more and 55% by mass or less and a proportion of the diglyceride of 1% by mass or more and 35% by mass or less with respect to the oil and fat components in the above-mentioned first composition are easily obtained.

Furthermore, it can be understood that repetitive use of the lipase is enabled as a result of the increased stability of the lipase.

[Examples 7 and 8 (esterification reaction and molecular distillation treatment)]

1) Esterification Reaction

The immobilized enzyme used in Example 1 was replaced with the immobilized enzyme prepared in Preparation Example 2, and an esterification reaction was conducted at the water content and reaction time described in Table 2, whereby compositions containing a DHA-containing glyceride (first compositions) were respectively obtained.

2) Molecular Distillation Treatment

The first compositions obtained in Examples 7 and 8 were each subjected to molecular distillation by using a short pass distillator (manufactured by KOBELCO Eco-Solutions Co., Ltd.) at a vacuum degree of 0.1Torr or less and a temperature of 80°C or more and 200°C or less, and other fatty acid ethyl esters including EPA ethyl ester were distilled off, whereby compositions containing a DHA-containing glyceride (second compositions) were respectively obtained.

The results of the component analysis of the first compositions and second compositions obtained in Examples 7 and 8 are shown in Table 2. In addition, the acid values of the above-mentioned first compositions and second composition were 12 or less.

[Table 2]

| | | Water content (% by mass) | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA / EPA | Acid value |
|---|---|---|---|---|---|---|---|---|---|---|---|---|
| Example 7 | First composition (after esterification reaction) | 0.5 | 24 | 73.2 | 4.2 | 10.0 | 11.2 | 25.4 | 14.8 | 35.1 | 2.4 | - |
| | Second composition (after molecular distillation) | | | 0.7 | 17.5 | 69.2 | 11.8 | 98.5 | 11.2 | 30.0 | 2.7 | - |
| Example 8 | First composition (after esterification reaction) | 2 | 24 | 75.6 | - | 7.5 | 16.8 | 24.3 | 9.6 | 32.5 | 3.4 | - |
| | Second composition (after molecular distillation) | | | 3.4 | 5.5 | 56.6 | 33.9 | 96.0 | 11.1 | 40.0 | 3.6 | 3.8 |

It can be understood from Table 2 that compositions containing a DHA-containing glyceride having an acid value of 12 or less can be obtained by that the water content in the reaction liquid of the above-mentioned esterification reaction is 0.4% by mass or more (Examples 7 and 8 first compositions and second compositions).

<Regarding First Compositions>

Furthermore, it can be understood that the first compositions obtained in Examples 7 and 8 are HA-containing glyceride-containing compositions each having an acid value of 12 or less, having a glyceride content of 20% by mass or more and 60% by mass or less with respect to the oil and fat components in the above-mentioned composition, and containing DHA and EPA as constituent fatty acids in the above-mentioned glyceride at the following mass ratio:

$$0.5 \leq (\mathrm{DHA} / \mathrm{EPA} \text{ in glycerides}) \leq 10.$$

It can be understood that compositions containing a DHA-containing glyceride each having a proportion of the DHA contained as a constituent fatty acid in the glycerides of the above-mentioned first compositions of 20% by mass or more and 90% by mass or less are easily obtained.
It can be understood that compositions containing a DHA-containing glyceride having a proportion of the monoglyceride of 1% by mass or more and 55% by mass or less and a proportion of the diglyceride of 1% by mass or more and 35% by mass or less with respect to the oil and fat components in the above-mentioned first composition are easily obtained.

<Second Composition>

Furthermore, it can be understood that the second compositions obtained in Examples 7 and 8 are HA-containing glyceride-containing compositions each having an acid value of 12 or less, having a glyceride content of 70% by mass or more and 100% by mass or less with respect to the oil and fat components in the above-mentioned composition, and containing DHA and EPA as constituent fatty acids in the above-mentioned glycerides at the following mass ratio:

$$0.5 \leq (\mathrm{DHA} / \mathrm{EPA} \text{ in glycerides}) \leq 10.$$

It can be understood that compositions containing a DHA-containing glyceride having a proportion of the DHA contained as a constituent fatty acid in the glycerides in the above-mentioned second composition of 20% by mass or more and 90% by mass or less are easily obtained.
It can be understood that compositions containing a DHA-containing glyceride having a proportion of the monoglyceride of 5% by mass or more and 45% by mass or less and a proportion of the diglyceride of 30% by mass or more and 90% by mass or less with respect to the oil and fat components in the above-mentioned second composition are easily obtained.
For the second composition of Example 7, the contents of the DHA and EPA as the constituent fatty acids contained in the respective glyceride fractions are shown in Table 3.

[Table 3]

| | EPA content in each glyceride (% by mass) | DHA content in each glyceride (% by mass) |
|---|---|---|
| Monoglyceride fraction | 11.3 | 36.6 |
| Diglyceride fraction | 12.1 | 41.1 |
| Triglyceride fraction | 11.9 | 31.0 |

It can be understood from Table 3 that a composition containing a DHA-containing glyceride having a DHA content contained in a monoglyceride of 15% by mass or more and 50% by mass or less, a DHA content contained in a diglyceride fraction of 20% by mass or more and 55% by mass or less, and a DHA content contained in a triglyceride fraction of 10% by mass or more and 45% by mass or less is easily obtained.

[Examples 9 to 12 (esterification reaction)]

An esterification reaction was conducted in a similar method to that of Example 1, except that the enzyme amount, ethanol amount and water content in the esterification reaction Example 1 were replaced with the enzyme amount, ethanol amount and water content described in Table 4, and component analysis was conducted. In addition, the compositions containing a DHA-containing glyceride obtained in Examples 9 to 12 each had an acid value of 12 or less.

[Table 4]

| | Use amount of enzyme (g) | Use amount of Ethanol | Water content (% by mass) | Reaction time (hour) | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA/EPA |
|---|---|---|---|---|---|---|---|---|---|---|---|---|
| Example 9 | 105 | Identical with the use amount of Example 1 | 2 | 2 | 14.4 | 29.4 | 36.0 | 20.2 | 85.6 | 18.8 | 14.9 | 0.8 |
| | | | | 4 | 39.8 | 14.3 | 27.3 | 18.7 | 60.3 | 18.3 | 20.3 | 1.1 |
| | | | | 6 | 56.1 | 7.5 | 16.8 | 19.7 | 44.0 | 17.5 | 26.0 | 1.5 |
| | | | | 8 | 52.9 | 4.0 | 8.4 | 22.4 | 34.8 | 17.3 | 29.0 | 1.7 |
| | | | | 24 | 75.5 | 3.6 | 0.6 | 18.1 | 22.3 | 5.8 | 38.7 | 6.7 |
| Example 10 | 210 | Identical with the use amount of Example 1 | 4 | 2 | 18.0 | 35.2 | 25.4 | 21.4 | 82.0 | 18.1 | 15.3 | 0.8 |
| | | | | 4 | 48.3 | 14.1 | | 36.8 | 50.9 | 17.7 | 22.3 | 1.3 |
| | | | | 6 | 72.5 | 0.0 | 14.9 | 12.6 | 27.5 | 16.6 | 35.7 | 2.1 |
| | | | | 8 | 65.2 | 4.0 | 8.4 | 22.4 | 34.8 | 17.5 | 28.9 | 1.7 |
| | | | | 24 | 82.0 | 3.3 | 0.4 | 10.3 | 14.0 | 18.4 | 59.9 | 3.3 |
| Example 11 | 210 | Added in 105 g at each of the timepoints of 0 h and 4 h after the initiation of the reaction | 4 | 2 | 47.3 | 13.0 | 20.7 | 19.0 | 52.7 | 18.1 | 22.2 | 1.2 |
| | | | | 4 | 46.8 | 14.8 | 19.9 | 17.4 | 52.1 | 17.9 | 21.6 | 1.2 |
| | | | | 6 | 61.5 | 4.5 | 12.8 | 21.3 | 38.6 | 17.1 | 27.5 | 1.6 |
| | | | | 8 | 63.4 | 2.9 | 7.0 | 26.8 | 36.7 | 17.6 | 28.7 | 1.6 |
| | | | | 24 | 79.8 | 4.0 | 0.6 | 13.5 | 18.1 | 5.8 | 34.6 | 6.0 |
| Example 12 | 210 | Added in 210 g at the timepoint of the initiation of the reaction | 0.5 | 2 | 17.8 | 10.5 | 30.4 | 41.3 | 82.2 | 20.0 | 15.6 | 0.8 |
| | | | | 4 | 20.6 | 3.7 | 26.6 | 49.1 | 79.4 | 19.7 | 16.0 | 0.8 |
| | | | | 6 | 24.7 | 2.1 | 21.6 | 51.5 | 75.2 | 19.2 | 16.6 | 0.9 |
| | | | | 8 | 31.2 | 0.8 | 19.5 | 48.6 | 68.9 | 19.0 | 17.8 | 0.9 |
| | | | | 24 | 54.4 | 0.0 | 10.2 | 35.4 | 45.6 | 18.8 | 22.8 | 1.2 |

It can be understood from Table 4 that compositions containing a DHA-containing glyceride having high purity and fine quality (first composition) can be obtained more efficiently by containing 0.1 parts by mass or more and 3.0 parts by mass or less of a lower alcohol with respect to 9.5 parts by mass of the above-mentioned raw material oil and fat in the reaction liquid of the above-mentioned esterification reaction (Examples 9 to 12).

<Regarding First Composition>

Specifically, it can be understood that the obtained first compositions are each a composition containing a DHA-containing glyceride having an acid value of 12 or less, a glyceride content of 20% by mass or more and 60% by mass or less with respect to the oil and fat component in the above-mentioned composition, and containing DHA and EPA as constituent fatty acids in the above-mentioned glycerides at the following mass ratio:

$$0.5 \leq (DHA / EPA \text{ in glycerides}) \leq 10.$$

It can be understood that a composition containing a DHA-containing glyceride having a proportion of the DHA contained as a constituent fatty acid in the glycerides of the above-mentioned first composition of 20% by mass or more and 90% by mass or less are easily obtained.

It can be understood that a composition containing a DHA-containing glyceride having a proportion of the monoglyceride of 1% by mass or more and 55% by mass or less and a proportion of the diglyceride of 1% by mass or more and 35% by mass or less with respect to the oil and fat components in the above-mentioned first compositions are easily obtained.

[Example 13 (esterification reaction, molecular distillation treatment and hydrolysis reaction)

1) Esterification reaction

1 kg of a purified fish oil (sardine oil: acid value: 0.3, triglyceride: 95% by mass, DHA content in fatty acid composition: 12% by mass) was put into a separable flask (volume: 3 L), and 52.5 g of ethanol was added.

The flask was mixed to allow the ethanol to homogeneously disperse in the fish oil. Then, 2.8 g of water (water content in reaction liquid: 0.24% by mass) was put into the flask, the mixture was stirred to allow the water to disperse in the fish oil-ethanol mixture, whereby a reaction liquid was prepared.

Subsequently, 100 g of the immobilized enzyme prepared in Preparation Example 1 was added, the air in the sample bottle was purged with nitrogen, and the sample was then reacted by using a stirrer at 150 rpm and 28°C for 21 hours to give a composition containing a DHA-containing glyceride (first composition).

Furthermore, 52.5 g of ethanol and 2.8 g of water were added to the reaction liquid at each of the timepoints of 2 and 4 hours from the initiation of the reaction, the sample bottle was purged with nitrogen, and an esterification reaction was conducted.

2) Molecular Distillation Treatment

The above-mentioned first composition was subjected to molecular distillation in a similar method to that of Examples 7 and 8 to distill off other fatty acid ethyl esters including EPA ethyl ester, whereby a composition containing a DHA-containing glyceride (Second Composition A) was obtained. The above-mentioned esterification reaction and molecular distillation treatment were repetitively conducted to give 1 kg of Second Composition A.

The above-mentioned second composition was appropriately mixed with a composition containing a DHA-containing glyceride having the composition shown in the following Composition 1 to give Second Composition B.

<Composition 1>

| | |
|---|---|
| Ethyl ester | 1% |
| Triglyceride | 18% |
| Diglyceride | 69% |
| Monoglyceride | 12% |
| Total | 100% |

3)-1 Hydrolysis Reaction

1 kg of the above-mentioned Second Composition B (acid value: 8.0) was put into a separable flask (volume: 3 L), and 333 g of water was added (water content with respect to raw material oil and fat: 33.3% by mass).

The flask was mixed to allow the water to homogeneously disperse, whereby a reaction liquid was prepared. A lipase OF (manufactured by Meito Sangyo Co., Ltd.) was then added so as to be 400,000 U (400 U/g with respect to raw material oil and fat), the atmospheric air in the sample bottle was purged with nitrogen, and the sample was reacted at 150 rpm and 40°C for 17 hours by using a stirrer to give a composition containing a DHA-containing glyceride.

3)-2 Molecular Distillation

The above-mentioned composition containing a DHA-containing glyceride was subjected to a molecular distillation treatment in a similar method to that of Examples 7 and 8 to give a third composition.

Component analysis was conducted on the first to third compositions by the above-mentioned method, and the results thereof are shown in Table 5.

[Table 5]

| | | | Esterification rate (% by mass) | Triglyceride (% by mass) | Diglyceride (% by mass) | Monoglyceride (% by mass) | Glyceride content (% by mass) | EPA content in glycerides (% by mass) | DHA content in glycerides (% by mass) | DHA /EPA | Acid value |
|---|---|---|---|---|---|---|---|---|---|---|---|
| Example 13 | First Composition | After esterification reaction | 78.0 | 1.0 | 11.0 | 10.0 | 22.0 | 17.5 | 27.8 | 1.6 | - |
| | Second Composition A | After molecular distillation | - | 25.0 | 49.0 | 26.0 | 100.0 | 16.6 | 279 | 1.7 | - |
| | Second Composition B | Mixed with composition containing a DHA-containing glyceride of Composition 1 | - | 44.0 | 43.0 | 13.0 | 100.0 | 14.5 | 28.7 | 2.0 | - |
| | Third Composition | Molecular distillation treatment after hydrolysis | - | 59.0 | 36.0 | 5.0 | 100.0 | 13.8 | 47.1 | 3.4 | .1.1 |

It can be understood from Table 5 that a composition containing a DHA-containing glyceride having high purity and fine quality having a proportion of the DHA contained as a constituent fatty acid in the glycerides in the obtained composition containing a DHA-containing glyceride of 30% by mass or more and 80% by mass or less (third composition) can be obtained more efficiently, by including a step of conducting a hydrolysis reaction by reacting a lipase in the presence of water with the composition containing a DHA-containing glyceride obtained by the step including an esterification reaction and a molecular distillation treatment reaction, and a step of further separating the glycerides and other components by molecular distillation (Example 13).

<Regarding Third Composition>

It can be understood that the above-mentioned composition containing a DHA-containing glyceride is specifically a composition containing a DHA-containing glyceride having an acid value of 0.1 or more and 5 or less, having a glyceride content with respect to the oil and fat components in the above-mentioned composition containing a DHA-containing glyceride of 80% by mass or more and 100% by mass or less, and contains DHA and EPA as constituent fatty acids in the above-mentioned glyceride at the following mass ratio:

$$2 \leq (DHA / EPA \text{ in glycerides}) \leq 6.$$

Furthermore, it can be understood that a composition containing a DHA-containing glyceride having a proportion of the monoglyceride of 0% or more and 20% by mass or less and a proportion of the diglyceride of 5% by mass or more and 50% by mass or less with respect to the oil and fat components in the above-mentioned composition containing a DHA-containing glyceride is easily obtained.

Furthermore, it can be understood that a composition containing a DHA-containing glyceride having a proportion of the triglyceride with respect to the oil and fat components in the above-mentioned composition containing a DHA-containing glyceride of 40% or more and 95% by mass or less is easily obtained.

[Example 14 (food composition: cookie)]

Cookies were prepared based on Formulation Table 1. Shortening and the composition containing a DHA-containing glyceride obtained in Example 7 (second composition) were put into a stirrer (Kitchen Aid manufactured by Kitchen Aid K5SS), mixed by a velocity controlling lever 6 for 1 minute until the mixture became creamy, powdered whole egg and sugar were added, and mixing was conducted.

Subsequently, pure water was gradually added to adjust the specific gravity to 0.8 g/mL, wheat flour and a baking powder that had been mixed in advance and sieved were then added, and the mixture was stirred for 30 seconds to prepare a dough. The obtained dough was let set in a refrigerator for 2 hours, extended to a thickness of about 3 to 5 mm, formed and baked in an oven at 180°C for 13 to 15 minutes to give cookies.

[Formulation Table 1]

| | |
|---|---|
| Wheat flour | 200 g |
| Baking powder | 1 g |
| Composition containing a DHA-containing glyceride (Example 7 second composition) | 1 g |
| Shortening | 120 g |
| Caster sugar | 80 g |
| Powdered whole egg | 12 g |
| Pure water | 24 g |
| Total | 438 g |

[Example 14 (soft capsule)]

Based on Formulation Table 2, a soft capsule was prepared by using the composition containing a DHA-containing glyceride obtained in Example 10 (second composition).

<Formulation Table 2>

| | |
|---|---|
| Composition containing a DHA-containing glyceride (Example 8 second composition) | 20% |
| Olive oil | 50% |

(continued)

| | |
|---|---|
| Beeswax | 10% |
| Middle chain fatty acid triglyceride | 10% |
| Emulsifier | 10% |
| Total | 100% |

**Claims**

1. A method for producing a composition containing a DHA-containing glyceride, comprising:

a step of obtaining a composition containing a DHA-containing glyceride, in which an esterification reaction is conducted by reacting a lipase with a raw material oil and fat containing highly unsaturated fatty acids as constituent fatty acids in the presence of water and a lower alcohol to give a composition containing a DHA-containing glyceride,
wherein a reaction liquid in the reaction has a water content of 0.4% by mass or more, and
wherein the composition containing a DHA-containing glyceride has an acid value of 12 or less.

2. The method for producing a composition containing a DHA-containing glyceride according to claim 1, wherein the reaction liquid contains 0.1 part by mass or more and 3.0 parts by mass or less of the lower alcohol with respect to 9.5 parts by mass of the raw material oil and fat.

3. The method for producing a composition containing a DHA-containing glyceride according to claim 1 or 2, wherein the proportion of the lower alcohol fatty acid esterified product with respect to the composition containing a DHA-containing glyceride is 40% by mass or more.

4. A composition containing a DHA-containing glyceride having an acid value of 12 or less, having a glyceride content of 20% by mass or more and 60% by mass or less with respect to the oil and fat components in the composition containing a DHA-containing glyceride, and containing DHA and EPA as constituent fatty acids in the glycerides at the following mass ratio:

$$0.5 \le (DHA / EPA \text{ in glycerides}) \le 10.$$

5. The composition containing a DHA-containing glyceride according to claim 4, wherein the proportion of the DHA contained as the constituent fatty acid in the glycerides of the composition containing a DHA-containing glyceride is 20% by mass or more and 90% by mass or less.

6. The composition containing a DHA-containing glyceride according to claim 4 or 5, wherein the composition containing a DHA-containing glyceride contains a monoglyceride at a ratio of 1% by mass or more and 55% by mass or less and a diglyceride at a ratio of 1% by mass or more and 35% by mass or less with respect to the oil and fat components in the composition containing a DHA-containing glyceride.

7. The method for producing a composition containing a DHA-containing glyceride according to any one of claims 1 to 3, wherein the composition obtained by the esterification reaction further contains a lower alcohol fatty acid ester, the method further comprises a step of separating a glyceride and other components by molecular distillation of the composition containing a DHA-containing glyceride, and wherein the glyceride content of the composition containing a DHA-containing glyceride is 70% by mass or more and 100% by mass or less.

8. A composition containing a DHA-containing glyceride having an acid value of 12 or less, having a glyceride content of 70% by mass or more and 100% by mass or less with respect to the oil and fat components in the composition containing a DHA-containing glyceride, and containing DHA and EPA as constituent fatty acids in the glycerides at the following mass ratio:

$$0.5 \leq (DHA / EPA \text{ in glycerides}) \leq 10.$$

9. The composition containing a DHA-containing glyceride according to claim 8, wherein the proportion of the DHA contained as the constituent fatty acid in the glycerides of the composition containing a DHA-containing glyceride is 20% by mass or more and 90% by mass or less.

10. The composition containing a DHA-containing glyceride according to claim 8 or 9, which contains a monoglyceride at a ratio of 5% by mass or more and 45% by mass or less and a diglyceride at a ratio of 30% by mass or more and 90% by mass or less with respect to the oil and fat components in the composition containing a DHA-containing glyceride.

11. The method for producing a composition containing a DHA-containing glyceride according to claim 7, wherein the method further comprises a step of conducting a hydrolysis reaction by reacting a lipase with the composition obtained by the molecular distillation treatment in the presence of water, and further separating a glyceride and other components by molecular distillation, and wherein the proportion of the DHA contained as the constituent fatty acid in the glycerides of the composition containing a DHA-containing glyceride is 30% by mass or more and 80% by mass or less.

12. A composition containing a DHA-containing glyceride having an acid value of 0.1 or more and 5 or less, having a glyceride content of 80% by mass or more and 100% by mass or less with respect to the oil and fat components in the composition containing a DHA-containing glyceride, and containing DHA and EPA as constituent fatty acids in the glycerides at the following mass ratio:

$$2 \leq (DHA / EPA \text{ in glycerides}) \leq 15.$$

13. The composition containing a DHA-containing glyceride according to claim 12, wherein the proportion of the monoglyceride is 0% or more and 20% by mass or less, and the proportion of the diglyceride is 5% by mass or more and 50% by mass or less with respect to the oil and fat components in the composition containing a DHA-containing glyceride.

14. The composition containing a DHA-containing glyceride according to claim 12 or 13, wherein the proportion of the triglyceride with respect to the oil and fat components in the composition containing a DHA-containing glyceride is 40% or more and 95% by mass or less.

15. A composition containing a DHA-containing glyceride obtained by the production method according to any one of claims 1 to 3, 7 and 11.

16. A method for producing a food composition, comprising a step of obtaining a food composition by using a composition containing a DHA-containing glyceride obtained by the production method according to any one of claims 1 to 3, 7 and 11, or the composition containing a DHA-containing glyceride according to any one of claims 4 to 6, 8 to 10 and 12 to 15.

17. A method for producing a capsule formulation, comprising a step of obtaining a capsule formulation by using a composition containing a DHA-containing glyceride obtained by the production method according to any one of claims 1 to 3, 7 and 11, or the composition containing a DHA-containing glyceride according to any one of claims 4 to 6, 8 to 10 and 12 to 15.

# Fig.1

```
                    ┌─────────────────┐
                    │      START      │
                    └─────────────────┘
                             │
                             ▼
        ┌──────────────────────────────────────────┐
        │   STEP 1  ESTERIFICATION REACTION         │
        │   (PRODUCTION OF FIRST COMPOSITION)       │
        └──────────────────────────────────────────┘
                             │
                             ▼
        ┌──────────────────────────────────────────┐
        │   STEP 2  MOLECULAR DISTILLATION          │
        │   (PRODUCTION OF SECOND COMPOSITION)      │
        └──────────────────────────────────────────┘
                             │
                             ▼
        ┌──────────────────────────────────────────┐
        │   STEP 3  MOLECULAR DISTILLATION          │
        │          AFTER HYDROLYZATION              │
        │   (PRODUCTION OF THIRD COMPOSITION)       │
        └──────────────────────────────────────────┘
                             │
                             ▼
                    ┌─────────────────┐
                    │       END       │
                    └─────────────────┘
```

## INTERNATIONAL SEARCH REPORT

| | International application No. |
|---|---|
| | PCT/JP2016/059770 |

**A. CLASSIFICATION OF SUBJECT MATTER**
*C12P7/64*(2006.01)i, *A23L33/12*(2016.01)i, *A61K9/48*(2006.01)i, *A61K31/232*(2006.01)i, *A61K47/14*(2006.01)i, *A61K47/44*(2006.01)i, *A61P3/02*(2006.01)i, *A61P3/06*(2006.01)i, *A61P7/02*(2006.01)i, *A61P25/28*(2006.01)i
According to International Patent Classification (IPC) or to both national classification and IPC

**B. FIELDS SEARCHED**

Minimum documentation searched (classification system followed by classification symbols)
C12P7/64, A23L33/12, A61K9/48, A61K31/232, A61K47/14, A61K47/44, A61P3/02, A61P3/06, A61P7/02, A61P25/28

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2009/017102 A1 (Nippon Suisan Kaisha, Ltd.), 05 February 2009 (05.02.2009), particularly, claims; paragraphs [0002], [0004], [0005], [0019], [0023] to [0026]; examples<br>& JP 5204776 B          & US 2010/0190220 A1 paragraphs [0002], [0004], [0005], [0023] to [0026]; examples<br>& EP 2172558 A1 | 1-17<br>1-17 |

☒ Further documents are listed in the continuation of Box C.   ☐ See patent family annex.

| | |
|---|---|
| *     Special categories of cited documents:<br>"A"   document defining the general state of the art which is not considered   to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 08 June 2016 (08.06.16) | 21 June 2016 (21.06.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2016/059770 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y | WO 2007/119811 A1 (Nippon Suisan Kaisha, Ltd.),<br>25 October 2007 (25.10.2007),<br>particularly, claims; paragraphs [0002],<br>[0004], [0005], [0011], [0017], [0018];<br>examples<br>& JP 5111363 B         & US 2009/0176284 A1<br>paragraphs [0002], [0004], [0005], [0018],<br>[0019]; examples<br>& EP 2006389 A2 | 1-10,15-17<br>1-17 |
| X<br>Y | JP 3-103499 A (Meito Sangyo Co., Ltd.),<br>30 April 1991 (30.04.1991),<br>particularly, claims; page 4, lower right<br>column, lines 5 to 9; examples<br>(Family: none) | 1-6,15-17<br>1-17 |
| A | JP 2009-153485 A (Maruha Nichiro Seafoods<br>Inc.),<br>16 July 2009 (16.07.2009),<br>(Family: none) | 1-17 |
| A | JP 2001-245686 A (Osaka-Shi),<br>11 September 2001 (11.09.2001),<br>(Family: none) | 1-17 |
| A | JP 7-51075 A (NOF Corp.),<br>28 February 1995 (28.02.1995),<br>(Family: none) | 1-17 |
| A | JP 58-165796 A (Asahi Denka Co., Ltd.),<br>30 September 1983 (30.09.1983),<br>(Family: none) | 1-17 |
| A | ZUYI, L. et al., Lipase-catalyzed alcoholysis<br>to concentrate the n-3 polyunsaturated fatty<br>acid of cod liver oil, Enzyme Microb. Technol.<br>1993, Vol.15, pp.601-6 | 1-17 |
| A | SHIMADA, Y. et al., Enrichment of Ethyl<br>Docosahexaenoate by Selective Alcoholysis with<br>Immobilized Rhizopus delemar Lipase, Journal of<br>Fermentation and Bioengineering, 1997, Vol.84,<br>No.2, p.138-43 | 1-17 |
| P,X | WO 2015/046436 A1 (Kewpie Corp.),<br>02 April 2015 (02.04.2015),<br>particularly, claims; examples<br>& JP 5753963 B | 1-10,15-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H787793 B

- JP 453031 A